# EUROPEAN PATENT APPLICATION

(11) **EP 4 230 244 A1**
(43) Date of publication of application: **23.08.2023**
(21) Application number: 22157655.6
(22) Date of filing: 21.02.2022
(51) Int. Cl.: A61M 16/04

(54) **HANDHELD TRACHEOSTOMY GUIDE AND TRACHEOSTOMY KIT**

(71) Applicant: innotrach ApS, 2770 Kastrup (DK)
(72) Inventor: HOFMAN ROSENKRANZ, Søren, 2300 København S (DK)
(74) Representative: Brann AB

(57) **Abstract**

A handheld tracheostomy guide (1) for use on a patient and a tracheostomy kit (100) are proposed. The tracheostomy guide comprises: a support body (10), having a proximal end (12) and an opposed distal end (14), and forming a front side (16) and a rear side (18), a first side (20), and an opposed second side (22), each extending in a direction from the proximal end (12) to the distal end (14), and connecting a front side (16) and a rear side (18), and wherein the combined first side (20) and second side (22) are configured for a pinch grip, such as a palmar or a lateral pinch grip. The support body forms a channel (24) for guiding a tracheostomy needle (80), or introducer needle, in a direction from the proximal end (12) to the distal end (14). The tracheostomy kit (100) comprises the abovementioned handheld tracheostomy guide (1) and a tracheostomy needle (80) positioned in the channel (24) of the support body (10) of the handheld tracheostomy guide (1).

## Description

### Technical field

The proposed technology relates generally to the field of devices for tracheostomy, especially created with percutaneous dilation technique. Further, the proposed technology relates specifically to the field of handheld tracheostomy guides. The technology also relates to a tracheostomy kit comprising a handheld tracheostomy guide and tracheostomy needle.

### Background

A tracheostomy is a medical procedure - either temporary or permanent - that involves creating an opening in the throat in order to place a tube into a person's windpipe.

The tube is inserted through a cut in the throat below the vocal cords. This allows air to enter the lungs. Breathing is then done through the tube, bypassing the mouth, nose, and throat. A tracheostomy can be created with an open surgical or a percutaneous dilation technique1 and can take place in the operating room or at the patient's bedside. The open technique involves dissection of the anterior pretracheal tissue and insertion of a tracheostomy tube under direct visualization. The percutaneous technique can be performed quickly and safely at the bedside with the use of a modified Seldinger technique and bronchoscopic guidance. This approach is associated with fewer bleeding complications than open tracheostomy and similar long-term morbidity.

A tracheostomy is performed for several reasons, all involving restricted airways. Breathing is done through the tracheostomy tube rather than through the nose and mouth. There are many reasons why sufficient air cannot get to the lungs. It may be done during an emergency when the airway is blocked. It could also be used when a disease or other problem makes normal breathing impossible or difficult. Thus, tracheostomies are performed due to a lack of air getting to the lungs.

A tracheostomy is usually done for one of three reasons: to bypass an obstructed upper airway; to clean and remove secretions from the airway; to more easily, and usually more safely, deliver oxygen to the lungs.

Conditions that may require a tracheostomy include but are not restricted to: anaphylaxis, congenital defects of the airway, burns of the airway from inhalation of corrosive material, cancer, chronic lung disease, coma, diaphragm dysfunction, facial burns or surgery, infection, injury to the larynx, laryngectomy, injury to the chest wall, need for prolonged respiratory or ventilator support, obstruction of the airway by a foreign body, obstructive sleep apnea, paralysis of the muscles used in swallowing severe throat or mouth injuries, tumours, vocal cord paralysis.

Tracheotomy and tracheostomy are two parts of the same surgical procedure which is done to facilitate breathing in a patient who is having issues related to respiration usually following trauma, foreign bodies, neurological problems, etc. The main difference between tracheotomy and tracheostomy is that tracheotomy is a surgical procedure which involves creating a direct airway incision made on the trachea whereas tracheostomy refers to an insertion of a tube through this airway. Additionally, the term "tracheotomy" refers to the incision into the trachea (windpipe) that forms a temporary or permanent opening, which is called a "tracheostomy". However, the terms are sometimes used interchangeably. A tracheostomy, the hole in the throat that the tube passes through, is commonly referred to as a "stoma".

In short, the procedure is usually performed as following. The patient is placed supine so that the throat is fully extended. The throat is palpated, and the anatomy is identified. Sometimes labelling the landmarks with a permanent marker is performed. The ideal location of the tracheostomy is regarded being between the 1st and 3rd tracheal ring. The introducer needle is used to puncture the anterior wall of the trachea. The guide wire is guided through the needle into the trachea. The needle is removed over the wire. The small tracheal dilator is advanced over the wire to dilate the tract. The small tracheal dilator is removed and advance the progressive dilator over the wire. The progressive dilator is removed, and an appropriately sized tracheostomy tube is inserted directly into the trachea over the wire. The tracheostomy tube is secured to the skin with sutures and tracheostomy tape.

The conventional way of providing tracheostomy has several disadvantages. First, when introducing the needle into the throat of a patient, the hand of the user may slip or tremble. Thus, the introducing of the needle requires certain physical characteristics of the user, such as manual strength and stability. Moreover, it may be difficult to secure the trachea between palpation and needle introduction since the skin may move in relation to the trachea. Further, it can be difficult to locate the centreline of the trachea, which is important when introducing the needle. Additionally, it can be difficult to palpate the tracheal rings, on, for example, obese patients. Second, when the needle is introduced into the throat, it must be held continuously by the user during the entire use of the needle. Thereby, the user has one hand only for operating other instruments. Therefore, the user usually needs assisting staff. Third, when the needle is introduced into the throat, the needle has to be held continuously at the exact same position to avoid posterior tracheal injury. This may be difficult for the user to perform, especially when a guide wire is fed into the needle.

The conventional kits are usually provided as separate parts, which has to be assembled before the tracheostomy procedure, which may be time-consuming and stressful. Moreover, as the instruments needed for tracheostomy procedure are usually individually sterile packaged, there is an expiring date of each of the packaging. Having several packaging with different expiring dates makes it difficult to overview and maintain the stock. Additionally, it is not time-efficient to open several packages. Further, the increased number of packaging leaves a larger environmental impact.

### Summary

The proposed technology aims at obviating the aforementioned disadvantages and failings of previously known tracheostomy guides, and at providing handheld tracheostomy guide and a tracheostomy kit that allows for a straight-forward and robust technology and which is capable of providing a tracheostomy in a short time and improved manner.

A primary object of the proposed technology is to provide handheld tracheostomy guide. It is a further object to decrease the tracheostomy procedure duration. Another object is to provide a guidance and support for a tracheostomy needle during the tracheostomy procedure, thereby avoiding posterior tracheal injury. Still another object is to provide a handheld tracheostomy guide that can be positioned accurately at the throat, is ergonomic and easy to use, and preferably requires fewer assisting personnel.

Another object of the proposed technology is to provide a tracheostomy kit comprising a handheld tracheostomy guide and a tracheostomy needle.

According to the proposed technology at least the primary object is attained by means of the initially defined handheld tracheostomy guide and tracheostomy kit having the features defined in the independent claims. Preferred alternative solutions of the proposed technology are further defined in the dependent claims.

According to a first aspect of the proposed technology, there is provided a handheld tracheostomy guide of the initially defined type. According to a second aspect of the proposed technology, there is provided a tracheostomy kit comprising a handheld tracheostomy guide and tracheostomy needle.

In a first aspect of the proposed technology a handheld tracheostomy guide for use on a patient is provided. The tracheostomy guide comprises:
a support body, having
   a proximal end and an opposed distal end, and forming
   a front side and a rear side,
   a first side, and an opposed second side, each extending in a direction from the proximal end to the distal end, and connecting the front side and the rear side, and wherein the combined first side and second side are configured for a pinch grip, such as a palmar or a lateral pinch grip, and
wherein the support body forms a channel for guiding a tracheostomy needle, or introducer needle, in a direction from the proximal end to the distal end.

According to a second aspect of the proposed technology, there is provided tracheostomy kit, or assembly, comprising a handheld tracheostomy guide and a tracheostomy needle positioned in a channel of the support body of the handheld tracheostomy guide.

The tracheostomy kit may be used as follows. A user grips the handheld tracheostomy guide and positions it at the throat of the patient. The tracheostomy needle is then guided, or pushed, through the channel of the support body to penetrate the throat of the patient. Preferably, the tracheostomy needle is locked, or secured, at the channel when it reaches a correct position, and is therefore hindered from moving in any direction. More preferably, the locking is provided at and/or by the channel of the support body. The tracheostomy procedure may then proceed as described above, i.e., feeding the guide wire into the needle, and removing the needle over the guide wire as the subsequent steps.

It is easier to manage the tracheostomy kit compared to the conventional tracheostomy equipment, as the kit may be a ready-to-use unit. This means that the necessary parts are provided and assembled for use, which is timesaving and may be especially advantageous in acute situations.

It is easier to transport and store the tracheostomy kit, and to overview the availability of the tracheostomy kits in stock, compared to the conventional tracheostomy equipment. This means that the tracheostomy kit makes it easier to supply the healthcare facilities with the equipment needed for the tracheostomy procedure as such. It is understood that conventional surgical equipment such as sterile cloths, compresses, medicaments, anaesthesia equipment, sutures does not have to be included in the tracheostomy kit but may be provided separately. As the kit may be packed as one unit in a packaging, only a single packaging per procedure has to be handled in the logistics chain, thereby making it easier to manage, transport, store and have an overview over the available equipment in stock. Moreover, having a single packaging reduces the environmental impact.

Alternatively, the parts of the tracheostomy kit may be packed in separate, or different, packaging. For example, the tracheostomy guide may be packed in a packaging different to a tracheostomy needle packaging. Packaging parts of the tracheostomy kit may result in a more compact packing of several packages in a transport or storage container. Thereby, a decreased environmental impact induced by transport may be achieved.

The tracheostomy guide is advantageous compared to conventional tracheostomy equipment in that it makes it possible to provide a tracheostomy in a convenient, secure, and straight-forward manner.

The proposed technology decreases the need of the manual strength and stability of the user required for the introducing of the needle by providing a support to the needle during the introducing step. Further, when the needle is introduced into the throat, the proposed tracheostomy guide holds the needle. Thereby, there is no need for the user to continuously hold the needle as it is supported by the tracheostomy guide. Thus, the user has two free hands for operating other instruments. Therefore, the conventional need of assisting staff is decreased. Additionally, the tracheostomy guide may prevent the needle from being pushed too far and thereby cause injury of the patient. Moreover, the proposed technology ensures that after the needle is introduced into the throat, the needle may be held continuously at the exact same depth to avoid posterior tracheal injury.

The user is herein defined as a surgeon, a physician, or other person having an adequate education, performing the tracheostomy procedure.

The expressions "proximal" and "distal" are to be understood in relation to the user while the tracheostomy guide is in use. Alternatively worded, "proximal" means nearest to the user, and "distal" means farthest from the user, while the tracheostomy guide is in use.

The distal direction is understood as the direction away from a user when the tracheostomy guide is in use. The proximal direction is opposite to the distal direction and is understood as the direction toward the user when the tracheostomy guide is in use.

It is understood that the user may use the tracheostomy guide directly, or manually, or indirectly, such as by using robot assistance.

The tracheostomy guide may be configured to be sterilized, for example, in autoclave. Therefore, the materials of the tracheostomy guide may, may be configured to resist high temperature and/or other sterilizing methods without being deformed, or in other way impacted such as to become inappropriate for usage. The tracheostomy guide may be configured to be reused.

It is understood that the support body is configured to support the needle. "To support" means here to hold up or serve as a foundation or prop. Thus, the support body serves as a prop for the needle. The support body may provide stability to the needle during the procedure. Further, the support body may provide support to the user during the tracheostomy procedure.

The support body may form a rigid structure. The tracheostomy guide may be made of a metal or metal alloy, or plastics such as polyvinyl chloride (PVC) or polyurethane. Preferably, the tracheostomy guide is manufactured of a material suitable for use in MRT.

The support body may be a monolithic, or solid, structure. This provides a ready-to-use product. Further, it provides for stability of the support body.

Alternatively, the support body is formed of several parts configured to be assembled prior to use. This provides for environmentally friendly transport and storage as parts of such reassembled tracheostomy guides are easier to stack on top of each other, and therefore require less space.

The support body may have a rough, or high-friction, exterior surface for gripping, providing a secure grip due to friction. Such rough surface may prevent the tracheostomy guide from rotating or gliding in the user's hand.

The support body has a proximal end and a distal end. The term "end" is understood to encompass "the part of an object that lies at the boundary of the object", or "a point that marks the extent of something".

It is understood that the distal end is configured for being in contact with a throat of a patient during use.

Preferably, the proximal end and the distal end have different dimensions for making the shape of the tracheostomy guide more ergonomic.

The support body forms a front side and a rear side. It is understood that the front side and the rear side each extend from the proximal end to the opposed distal end.

The support body forms the first side and the opposed second side. The first side and the opposed second side each extends in a direction from the proximal end to the distal end. The first side and the second side connect a front side and a rear side. Thus, the combined first side and second side provide a support for a pinch grip. The expression "pinch" is understood as to squeeze between the finger and thumb or between the jaws of an instrument. A palmar pinch grip is understood as a pinch between the palm facing sides of the thumb and the index or middle finger, usually between the distal phalanx of the thumb and the distal phalanx of the index or middle finger. A lateral pinch is understood as a pinch between the thumb and the thumb facing side, or radial side, of the index finger, usually between the distal phalanx of the thumb and the intermediate or proximal phalanx of the index finger.

It is understood that the channel is elongated and straight. This provides for an easy insertion of the needle. It is further understood that the channel has a central axis.

Preferably, the channel is positioned at the centre between the first side and the second side, or the channel bisects the support body. This provides a more accurate positioning.

The channel may extend along the front side from the proximal end to the distal end.

The channel may be aligned with the front side. The front side. This provides for a straight-forward manner of tracheostomy procedure as it makes it easier to the user to identify the position of the needle relative to the support body and the direction of the needle at insertion. The complete channel may be exposed at the front side.

The support body may be manufactured of a transparent or translucent material. Preferably, the channel may have sections manufactured of a transparent material. This provides for the visual inspection of the channel and the needle within. In this way, it is easy to identify the position of the needle in the channel or to read the markings on the needle.

The channel may comprise an outer side portion opposite, or relative, to the front side. The outer side portion may be releasably attached to the front side, for example by a click fit. This provides for a possibility of the inspection of the needle by disconnecting the outer side portion from the tracheostomy guide, or for the tracheostomy guide to be removed with the needle inserted into the trachea. Moreover, this provides for a better cleaning and/or disinfection of the tracheostomy guide if it is reused. Further, it provides for more environmentally friendly transport and storage as the reassembled tracheostomy guides are easier to stack on top of each other and fill less space. Moreover, the possibility of reassembling provides for a change in the transverse dimension of the channel. Thus, it is possible to use different thickness of needle with the tracheostomy guide. Thereby, the tracheostomy guide is versatile.

The tracheostomy guide or the tracheostomy kit may further comprise a position indicator indicating the position of the needle relative to the support body, thus allowing a user to position the tracheostomy needle in a predetermined position. For example, the position indicator may comprise a first marking on the support body and a second marking on the needle, wherein the markings are aligned at the predetermined position.

It is understood that the channel is configured for encompassing, or holding, or guiding a tracheostomy needle. The channel preferably has a circular cross section. More preferably, the channel has an inner surface having a geometry, or shape, conforming to the shape of the needle. The inner surface of the channel is preferably smooth for decreasing the friction when inserting the tracheostomy needle in the channel.

The term "needle" is used for a tracheostomy needle, or introducer needle, throughout these specifications.

The needle may be releasably supported by the support body. The needle may be configured to slide along the support body, in the channel.

It is understood that the needle may be sufficiently stiff in order to penetrate the tissue. It is further understood that the needle has a sharp end for penetrating the tissue. The other end of the needle may have an interface, or a connector, for receiving or connecting to additional equipment, such as a tube or a syringe. The additional equipment may be a guide wire and the interface may be a funnel for receiving the guide wire into the needle. The needle may have smooth surface, which contributes to reduced tissue damage in use.

The needle may be manufactured, partly or entirely, from steel, such as stainless steel or high carbon steel, or from a titanium alloy. The needle may be manufactured of a material suitable for use in MRT.

It is understood that the expression "kit" has a common meaning of a set of separate and separable elements that are assembled into a complete structure.

The channel may be further configured for guiding a tracheostomy dilator. This means that the transverse dimension, or width, of the channel is suitable for receiving a dilator.

The channel may have a proximal channel portion and a distal channel portion. The width, or transverse dimension of the proximal channel portion, may be greater than the width, or transverse dimension of the distal channel portion. This is advantageous as the channel may receive a needle having different width along its length. Alternatively or additionally, the needle may comprise an external part, such as a connector or funnel, and the proximal channel portion may be configured to receive the connector.

The channel may comprise a needle lock mechanism configured to lock, preferably releasably lock, the tracheostomy needle, to the support body. For example, this may be achieved by means of a click-fit or bayonet mount. This is advantageous as it provides for the needle staying in the correct position throughout the tracheostomy procedure. Moreover, the user does not need to hold the needle throughout the tracheostomy procedure.

The channel may have a cross section with a wide portion allowing a movement of the needle along the channel, and a narrow portion configured to engage with the needle and preventing a movement of the needle along the channel. The wide portion and the narrow portion are interconnected allowing the needle to transition from the wide portion to the narrow portion by transverse movement of the needle. For example, the channel may have a transverse cross-section with a wide portion having a width, or transverse dimension, being larger than the width, or transverse dimension of the tracheostomy needle, and a narrow portion having a width, or transverse dimension, being equal to or smaller than the width, or transverse dimension of the tracheostomy needle.

The presence of the cross section with the wide portion and the narrow portion is advantageous as it provides for a possibility of an easy introduction of the tracheostomy needle in the channel through the wide portion and a possibility of stabilising and supporting the tracheostomy needle in the channel in the narrow portion. In this way, the narrow portions act as a lock mechanism that secures or locks the tracheostomy needle when moving it from the wide portion to the narrow portion. Preferably, the tracheostomy needle may be locked in the narrow portion by click-fit.

The channel may comprise an open portion in which the needle guided by the channel is exposed on the front side of the support body.

It is understood that the cover provided at, or by, the portion may be incomplete. Worded differently, the cross-section of the open portion does not have a form of a ring but is preferably C-shaped or U-shaped. This means that the open portion does not completely enclose a tracheostomy needle positioned thein.

It is understood that the open portion may extend along at least one part of the channel. A portion of a tracheostomy needle is thereby exposed for visual inspection and/or manual operation. The visual inspection of the needle may involve inspection of markings on the needle or its progress in the channel. Examples of manual operation are wiping away or suction of blood from the needle or adding medicaments.

The open portion may be located at the proximal end. Preferably, the open portion is a portion of the channel that extends from the proximal end toward the distal end. This allows for receiving a connecter attached to a needle and having a larger width, or transversal dimensions, than the needle. Thus, the open portion provides space for the connector.

The open portion may be located at the distal end. Thus, the channel may comprise the open portion extending from the distal end toward the proximal end. This allows for inspection of the needle at the distal end. Thus, it possible for the user to see when the needle approaches and punctures the throat of the patient. Further, it provides a possibility of manipulating the needle at the distal end.

It is understood that the channel may have an open portion both at the distal and at the proximal end.

The channel may comprise at least a covered, or closed, portion for covering the tracheostomy needle. It is understood that the expression "closed" means that the portion is closed on its sides.

At the covered portion, the cross-section of the channel forms a ring. This way, the channel surrounds the needle in the covered portion. Alternatively, the complete channel may surround the needle. This provides for an increased support for the needle.

Worded differently, at the covered portion no inspection of the content of the channel is possible in a transverse direction, unless the channel, or portion, is made of a transparent material. Thus, the covered portion of the channel is configured for covering a portion of the tracheostomy needle positioned therein.

It is understood that the channel may comprise at least an open portion and at least a covered portion. For example, the channel may have a first open portion extending from the proximal end in a direction toward the distal end, a second open portion extending from the distal end in direction toward the proximal end, and a covered portion interconnecting the open portions. Hence, the channel may have an open portion at each end and a closed portion in-between. The combination of an open portion and a covered portion provides for stability and a possibility of visual inspection and manipulation of the tracheostomy needle. Further, it offers a possibility of positioning and manipulation of a connector of the tracheostomy needle.

The support body may comprise a tubular portion at the distal end. The tubular portion may form at least a part of the channel.

It is understood that the tubular portion extends from the distal end in toward the proximal end.

At the tubular portion, the cross-section channel forms a ring. This means that at the tubular portion, no inspection of the content of the channel is possible in a transverse direction, unless the channel is made of a transparent material. Thus, the tubular portion or the channel is configured for covering a portion of the tracheostomy needle positioned therein.

The tubular portion may connect to an open portion of the channel. The provision of both an open portion and a tubular portion provides a combination of stability and possibility of visual inspection and manipulation of the tracheostomy guide. Further, it offers a possibility of positioning and manipulation of a connector of the tracheostomy guide at the open portion.

The distal end of the support body may comprise a contact edge for contacting the throat of the patient. The contact edge is preferably concave. The concave form of the distal may conform to the throat of a patient, making positioning of the guide easier. Additionally, the concave distal end helps to secure and fixate the trachea and makes it easier to assess the centreline. It is understood that the contact edge is blunt.

The first side and the second side of the support body may converge toward the distal end. The converging first and second sides improves a palmar pinch. Worded differently, the converging results in tapered support body. This way, the support body is more secure to grip and hold.

Each of the first side and the second side may have a distal concave portion. Formed this way, the support body is more secure to grip, such as by lateral pinch grip. Further, it allows for an ergonomic pinch grip. Additionally, the converging allows for pulling the support body away from the throat of the patient when there is no longer need for the tracheostomy guide.

The contact edge may interconnect the first side and the second side.

The front side and the rear side may converge toward the distal end. The converging front side and rear side provide for an exact positioning of the tracheostomy guide at the throat of the patient.

The contact edge may interconnect the front side and the rear side. This has the effect of providing a sharp profile at the distal edge allowing for a close and exact positioning. The support body may form a blade at the distal end, wherein the edge is on the blade.

Each of the first side and the second side may have a proximal concave portion or friction surface. Preferably, the proximal concave portion is positioned closer to the proximal end than to the distal end. Effectively, each proximal concave portion forms a cut out in the support body. The cut outs allow for better gripping due to the friction. Especially, the cut outs provide support for a pinch grip, such as a palmar or a lateral pinch grip. The cut outs provide further friction while the user holds, or grips, the support body, thereby preventing the support body from slipping out of the hand of the user. Additionally, the cut outs allow for pushing in direction toward the throat of the patient.

The support body may have a manual and/or mechanical interface. Preferably, it may be on the rear side and at the proximal end. It is understood that the interface is for manipulating the tracheostomy guide. For example, the manual and/or mechanical interface may be used for connecting to a surgical instrument, surgical apparatus or a robotic arm. The manual and/or mechanical interface may provide a connection by means of, for example, a hook grasp, or a handle for a hook grasp.

The interface may be a protrusion extending from the rear side. The protrusion may form a sector of a ring or a complete ring. The protrusion may be configured for receiving a finger of the user. The ring may be oriented to receive the finger in a direction from the proximal end to the distal end.

It is understood that the expression "complete ring" means that no side opening is provided. It is further understood that the expression "sector" means that the ring has a side opening. For example, a "a sector" may correspond a sector with an angle of 315°.

It is further understood that the ring has a central axis.

The interface makes the improves the manipulation of the tracheostomy guide by providing further holding positions. For example, it can be held as a syringe. This means that the user may position a thumb at the protrusion, and index and middle finger at the first respective second side. This provides for an ergonomic and secure grip.

The protrusion may form a hole. The hole may define a cylinder. It is understood that the cylinder has a central axis. The hole may be a blind hole or a through hole. The hole may be configured for receiving a finger of a user, or an external instrument, or a robotic arm, in a direction from the proximal end to the distal end.

This improves the grip of a user. For example, the user may position a thumb in the hole, and index and middle finger at the first respective second side. This provides for an ergonomic and secure grip. Moreover, it provides for a more stable grip of the tracheostomy guide during positioning.

The ring has a central axis, which may be oriented at an angle in range of 15-60° relative to the central axis of channel. Preferably, the angle is in the range of 20-55°, 25-55°, 30-50°, 35-45°. Most preferably, the angle is at 21°. It has been found that such angles provide for a tracheostomy guide that can be accurately positioned and oriented in a single hand grip.

The central axis of the channel and the central axis of the ring may converge toward distal end.

The central axis of the channel and the central axis of the ring may be coplanar. The above relationships between the central axis of the channel and the central axis of the ring may also apply to the central axis of the channel and the central axis of the abovementioned cylinder defined by the hole formed by the protrusion.

The handheld tracheostomy may further comprise a tubular body. It is understood that the tubular body has a central axis. The tubular body may be attached to the support body. preferably to the protrusion. Preferably, the tubular body is releasably attached to the support body.

It is understood that the tubular body has a rear end and a forward end. It is further understood that the rear end of the tubular body is the end which is the most proximal end of the tubular body during use. Worded differently, the rear end faces the user.

The tubular body may have an aperture at the rear end. It is understood that the aperture faces the user during use of the tracheostomy guide.

The tubular body may be configured for receiving a finger of the user via the aperture. This means that the dimensions of the aperture and the tubular body are adapted for receiving a finger. The tubular body makes provides additional stability and further holding positions of the tracheostomy guide. For example, it can be held as a syringe. This means that the user may position a thumb in the tubular body, and index and middle finger at the first respective second side. This provides for an ergonomic and secure grip.

The tubular body may be inserted in the abovementioned hole in the protrusion. The tubular body may extend out from the hole in the protrusion in a direction toward the user. The tubular body may conform the hole in the protrusion, thus providing a secure mounting.

The tubular body may be releasably attached to the support body. Preferably, the tubular body is configured to be inserted in the hole formed by the protrusion. The tubular body may be configured to be locked to the support body, for example, by screwing, or click-fit, or an additional locking mechanism.

The tubular body may be cylindrical. The tubular body aperture may be circular.

The tubular body may be manufactured of the same material as the support body. The tubular body may be configured to be sterilized, for example, in autoclave. Alternatively, the tubular body may be configured to be disposable.

Alternatively, the tubular body may form a blind hole. The tubular body aperture may have a bottom at the forward end. The bottom may form a bottom aperture. The aperture may be used for anchoring a sheath, preferably a silicone sheath, which is used for lining the aperture of the tubular body. Alternatively, a lining of elastic foam may be used without anchoring.

The tubular body may be configured for being attached to an external instrument, such as a robot arm during robot assisted surgery, or to a connector. The tubular body may comprise an interface for connecting to the external instrument. The tubular body may be connected to an external instrument by means of, for example, click-fit or by screwing.

The hole formed by the protrusion may have a narrow portion engaging the rear end of the tubular body preventing passage of the tubular body through the hole. This provides for further stability and security of the tracheostomy guide.

Preferably, the tubular body may be releasably locked in position to the narrow portion by, for example, click-fit or threads. Preferably, the tubular body may form an indentation for fitting at the narrow portion. The narrow portion provides further stability and security when using the tracheostomy guide. The central axis of the tubular body may be oriented at an angle in range of 15-60° relative to the central axis of channel. Preferably, the angle is in the range of 20-55°, 25-55°, 30-50°, 35-45°. Most preferably, the angle is 21°. Such angle provides for a tracheostomy that can be positioned more accurately at the throat of the patient in a single hand grip.

Additionally, the central axis of the tubular body may be parallel to the central axis of the ring.

The central axis of the channel and the central axis of the tubular body may converge toward distal end.

The central axis of the channel and the central axis of tubular body may be coplanar.

The tracheostomy guide may further comprise a lock for releasably locking the support body and the tubular body. This provides for further stability and security of the tracheostomy guide. The tubular body is prevented from moving in the support body. Moreover, the connection between the tubular body and the support body provided by the lock provides a possibility of operating the tracheostomy guide by gripping only one of the tubular body and the support body. Additionally, the connection prevents the tubular body from dismounting from the support body during use.

The lock may comprise a locking member pivotably connected to the tubular body and configured to cooperate with support body. This means that the locking member may pivot relative to the tubular body.

It is understood that the locking member has a pivot axis, preferably perpendicular to central axis of the hole in the protrusion or the tubular body, wherein the locking member is configured to be pivoted around the pivot axis for releasing the tubular body from the support body.

The lock may have a manual lock interface by which a user can manipulate the lock and cause the locking member to pivot, preferably by pulling the lock interface away from the base support.

The tubular body may form a first cut out cooperating with the locking member to pivotably connect the locking member to the tubular body. The rear side of the support body may form a second cut out cooperating with the locking member to releasably lock the locking member to the support body. It is understood that the cut outs are dimensioned to receive the locking member. It is further understood that the first cut out is configured to allow for the pivoting of the locking member. The second cut out is dimensioned to allow the locking member and the thereto connected tubular body to be released, from the support body.

The dimensions of the locking member may be such that, when pivoted, the lock may be extracted, preferably while attached, or connected, to the tubular body, in a proximal direction through the hole.

The locking member may be releasably connected to the support body. For example, the second cut out may extend to the side of the tubular body and the locking member may be slidable along the cut out. The locking member may be releasably connected to the tubular body. The locking member may be configured to slide out of the tubular body along the pivot axis.

### Brief description of the drawings

A more complete understanding of the abovementioned and other features and advantages of the proposed technology will be apparent from the following detailed description of preferred embodiments in conjunction with the appended drawings, wherein:
Figures 1A - 1C schematically illustrate an embodiment of a tracheostomy guide.
Figure 1D schematically illustrates an embodiment of a tracheostomy kit having a tracheostomy guide and a needle.
Figure 1E schematically illustrates a channel with a needle lock mechanism.
Figure 2 schematically illustrates an embodiment of a tracheostomy guide having a tubular portion.
Figures 3A and 3B schematically illustrate an embodiment of a tracheostomy guide having a tubular body.
Figures 4A and 4B schematically illustrate two alternative embodiments embodiment tubular body.
Figures 5A and 5C schematically illustrate an embodiment of a tracheostomy guide having a lock.

### Description of the drawings

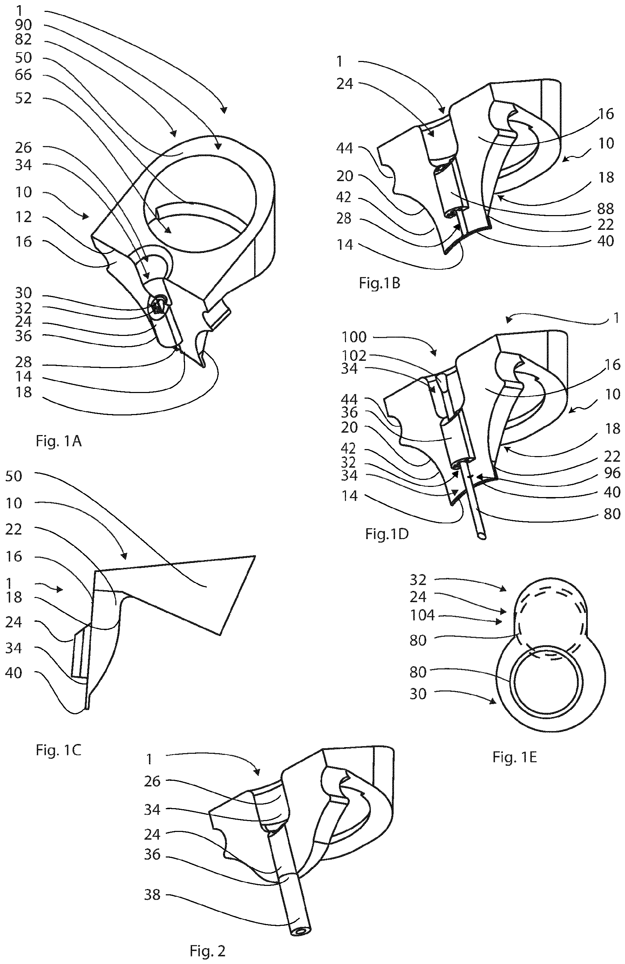

**Figures 1A** - **1C** schematically illustrate an embodiment of a tracheostomy guide 1. The tracheostomy guide is disposable and has a monolithic rigid support body 10 made of transparent polyvinyl chloride PVC. The support body 10 has a rough exterior surface for secure grip. The support body 10 has a proximal end 12 and an opposed distal end 14. When in use, the distal end 14 is in contact with the throat of the patient. The support body 10 forms a front side 16 and a rear side 18 extending between the proximal end 12 and the distal end 14. Here, the front side 16 and the rear side 18 converge toward the distal end 14 and are interconnected at a blunt concave contact edge 40 that conforms to the throat of a patient. Further, the support body 10 forms a first side 20 and an opposed second side 22 converging toward the distal end 14. Each of the first side 20 and the second side 22 has a distal concave portion 42 providing an ergonomic grip when pulling the support body away from the throat of the patient. Additionally, the first side 20 and the second side 22 each have a proximal concave portion 44 forming of a cut out in the support body 10 positioned closer to the proximal end 12 than to the distal end 14. The cut outs provide support for a pinch grip. Thus, the front side 16 and the rear side 18 extend between the first side 20 and the second side 22. Thereby, a support for a pinch grip and a surface to be in contact with the throat of the patient are provided.

The support body 10 has a manual and/or mechanical interface 82 in the form of a cylindrical through hole 52 in a protrusion 50, thus forming a ring on the rear side 18 and at the proximal end 12 of the support body 10. The through hole 52, and in extension the ring, has a central axis 84. The interface can be used for connecting to a surgical instrument or for placing a finger of the user in it. The user may position a thumb in the hole 52, and index and middle finger at the first respective second sides 20,22.

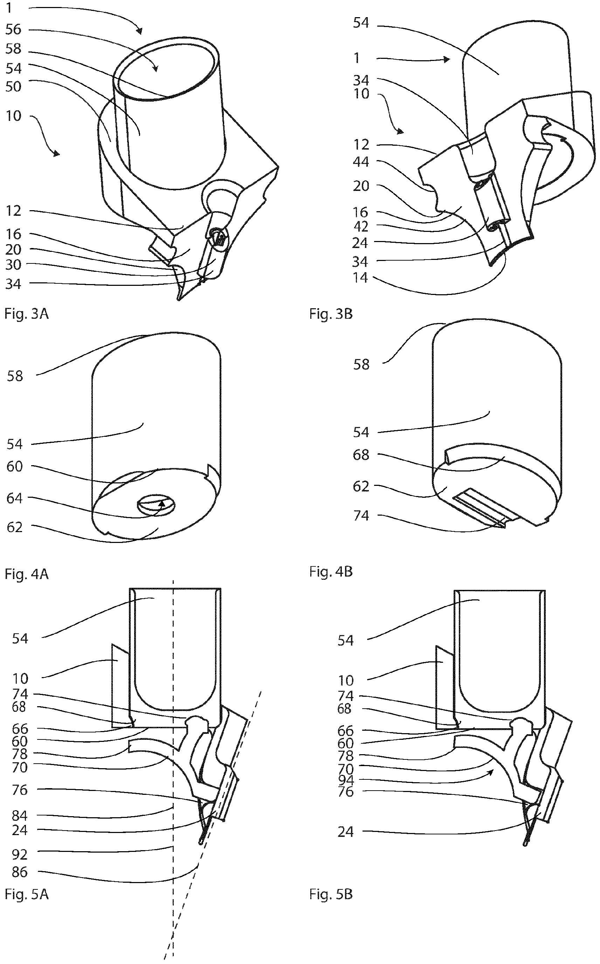

The support body 10 forms an elongated and straight channel 24 having a central axis 86 (see **Figure 5A**) and a smooth inner surface. The channel 24 is positioned at an indentation of the front side 16, and at an equally long distance from the first side 20 as from the second side 22. The channel 24 is parallel to a planar front surface of the front side 16. In this embodiment, the channel 24 has an outer side portion 88 relative to the front side 16, which is attached to the front side 16 by click fit (not shown). The channel 24 has a channel secure mechanism for securing that a tracheostomy needle is at a correct position (see **Figure 1D**). The channel 24 has dimensions for receiving a needle. Here, the channel has a proximal channel portion 26 which is wider than a distal channel portion 28. Thereby, the support body 10 is dimensioned for receiving a needle with a connector. Further, the channel 24 has a needle lock mechanism 104 for which locks the needle 80 to the support body 10 by friction. **Figure 1E** illustrates an example of a cross section of a channel 24. The cross section has a wide portion 30 interconnected to a narrow portion 32. The needle is allowed to transition from the wide portion 30 to the narrow portion 32 and be held by friction fit by the narrow portion 32. The needle 80 can slide along the wide portion 30 and is engaged by the channel in the narrow portion 32, thereby securing the needle 80 in the narrow portion 32.

The channel 24 has an open portion 34, which is C-shaped in cross section and extends from the proximal end 12 in the direction toward the distal end 14. Thus, the needle may be inspected at the open portion. The channel 24 has an additional open portion 34 extending from the distal end 14 to the proximal end 12, which allows receiving of a needle with a connector.

Further, the channel 24 has a covered portion 36 where the cross section of the channel 24 forms a ring. The covered portion 36 interconnects the two open portions 34. Thus, the covered portion 36 and the two open portions 34 are aligned and form the channel 34. It is thus possible to manipulate the needle at the proximal and at the distal channel portions 26, 28, but not at the covered portion 36.

The central axis 84 of the ring 90 is oriented at an angle of approximately 21° relative to the central axis 86 of channel 24. Thus, the central axis 86 of the channel 24 and the central axis 84 of the ring 90 converge toward distal end 14 and cross each other.

**Figure 2** schematically illustrates an embodiment of a tracheostomy guide 1. This alternative embodiment differs from the embodiment illustrated in Fig. 1A-1C in that the support body 10 has a transparent tubular portion 38 at the distal end 14 for covering the needle. Additionally, the channel 24 has a single open portion 34. Another difference is that the support body does not have a contact edge 40 at the distal end 14. Instead, the tubular portion 38 contacts the throat of the patient during use. The tubular portion 38 forms a part of the channel 24, and its cross section forms a ring. Thus, the tubular portion 38 together with the covered portion 36 and the open portion 34 the channel 34 form together the channel 24.

**Figures 3A and 3B** schematically illustrate an embodiment of a tracheostomy guide 1 having a disposable cylindric tubular body 54 with a central axis and manufactured of PVC. The tubular body 54 is inserted in the hole 52 and attached to the protrusion 50 of the support body 10 by click-fit (not shown). The tubular body 54 has a rear end 58 which extends out of the protrusion 50 and a forward end 60 inserted in the hole 52. Thereby, the tubular body 54 may be removed from the support body 10 by gripping the tubular body at the rear end 58. The user may place a finger in the tubular body through a cylindrical tubular body aperture 56, at the rear end 58. As illustrated in the figures, the tubular body 54 is oriented such that the finger is inserted in the tubular body aperture 56 in direction toward the throat of the patient. Thus, the tubular body may be held as a syringe by placing a thumb at the tubular body 54, and index and middle finger at the first respective second side 20,22.

Here, the central axis 92 of the tubular body 54 is colinear with the ring 90. Therefore, the central axis 92 of the of the tubular body 54 is oriented at an angle of approximately 21° relative to the central axis of channel 24. Thus, the central axis 86 of the channel 24 and the central axis 92 of the of the tubular body 54 converge toward distal end 14 and cross each other. Furthermore, the two axes 86 and 92 are coplanar.

**Figures 4A and 4B** schematically illustrate two alternative embodiments of a cylindrical tubular body 54 having a bottom 62 at the forward end 60 with a bottom aperture 64. The tubular body 54 can be attached to an external instrument by treads at the tubular body aperture 56 (not shown). The hole 52 formed by the protrusion 50 has a narrow portion 66 engaging the rear end 58 of the tubular body 54 which prevents passage of the tubular body 54 through the hole 52 (see **Figure 1A****).** The tubular body has indentation 68 at the forward end 60 for fitting at the narrow portion 66. Thereby, the tubular body 54 is prevented from passing through the support body 10.

**Figures 5A and 5B** schematically illustrate an embodiment of a tracheostomy guide 1 with a lock 94 that locks the tubular body 10 to the tubular body 54. The lock has a locking member 70 which is inserted in a first cut out 74 of the tubular body 54 and in a second cut out 76 of the support body 54. The difference between Figure 5A and 5B is that in the latter the locking member 70 is pivoted to release the locking member 70 from the support body 10. The lock 94 has a trigger-like lock interface 78, which is pulled for pivoting the locking member around a pivot axis, and thereby releasing the tubular body 54 from the support body 10.

The first cut out 74 extends to the side of the tubular body 54 and the locking member70 can slide along the first cut out 74 when it does not engage the support body 10. This way, the locking member 70 can be removed from the tubular body 52. The first cut out 74 has portions with stepper surface engaging the first cut out 74 so that the lock member is pivoted in the first cut out 74 in steps. It is also illustrated that the locking member 70 is prevented from being released from the tubular body 54 by pivoting.

The second cut out 76 has dimensions that allow the locking member 70 to slide out of the support body 10 when pivoted.

Thus, the first and the second cut outs 74, 76 and the locking member 70 together form the lock.

The tracheostomy guide 1 as described above may be used together with a needle 80, thereby forming a kit 100. The kit 100 is illustrated in Fig. 1D. A needle 80 is inserted in the channel 24 of the tracheostomy guide 1, as described in connection to Fig. 1B. The needle is enclosed in the covered portion 36 and exposed at the open portions 34 of the channel 24. The needle 80 is in the narrow portion 32 of the channel 24, and, thus, locked in position. A connector 102 is positioned within the proximal channel portion 26. A cutting edge of the needle 80 is positioned below the contact edge 40. It is thus understood that the tracheostomy kit 100 as presented in Fig. 1D is in use, and the needle 80 is illustrated in a position during or after the penetration. The tracheostomy kit 100 or has a position indicator 96 which indicates the position of the needle 80 relative to the support body 10. Here, the position indicator 96 is composed of a first marking on the support body 10 and a second marking on the needle 80. The markings are aligned showing the user that the needle has reached a desired position.

Item list
- 1: a handheld tracheostomy guide
- 10: a support body
- 12: a proximal end
- 14: a distal end
- 16: a front side
- 18: a rear side
- 20: a first side
- 22: a second side
- 24: a channel
- 26: a proximal channel portion
- 28: a distal channel portion
- 30: a wide portion of the channel
- 32: a narrow portion of the channel
- 34: an open portion of the channel
- 36: a covered or closed, portion
- 38: a tubular portion at the distal end
- 40: a contact edge
- 42: a distal concave portion
- 44: a proximal concave portion or friction surface
- 50: a protrusion
- 52: a hole in the protrusion
- 54: a tubular body
- 56: a tubular body aperture
- 58: a rear end of the tubular body
- 60: a forward end of the tubular body
- 62: a bottom
- 64: a bottom aperture
- 66: a narrow portion
- 68: an indentation of the support body
- 70: a locking member
- 74: a first cut out
- 76: a second cut out
- 78: a lock interface
- 80: a needle
- 82: a manual and/or mechanical interface
- 84: a central axis of the ring
- 86: a central axis of the channel
- 88: an outer side portion
- 90: a ring
- 92: a central axis of the tubular body
- 94: a lock
- 96: a position indicator
- 100: a kit
- 102: a connector
- 104: a needle lock mechanism

## Claims

1. A handheld tracheostomy guide (1) for use on a patient comprising:
a support body (10), having
a proximal end (12) and an opposed distal end (14), and forming
a front side (16) and a rear side (18),
a first side (20), and an opposed second side (22), each extending in a direction from the proximal end (12) to the distal end (14), and connecting the front side (16) and the rear side (18), and wherein the combined first side (20) and second side (22) are configured for a pinch grip, such as a palmar or a lateral pinch grip, and
wherein the support body (10) forms a channel (24) for guiding a tracheostomy needle (80), or introducer needle, in a direction from the proximal end (12) to the distal end (14) .

2. The handheld tracheostomy guide (1) according to claim 1 wherein the channel (24) is further configured for guiding a tracheostomy dilator.

3. The handheld tracheostomy guide (1) according to claim 1 or 2, wherein the channel (24) has a proximal channel portion (26) and a distal channel portion (28), wherein the width, or transverse dimension of the proximal channel portion (26) is greater than the width, or transverse dimension of the distal channel portion (28).

4. The handheld tracheostomy guide (1) according to any of the proceeding claims, wherein the channel (24) comprises a wide portion (30) allowing a movement of the needle along the channel (24), and a narrow portion (32) configured to engage with the needle (80) and preventing a movement of the needle (80) along the channel (24), and
wherein the wide portion (30) and the narrow portion (32) are interconnected allowing the needle (80) to transition from the wide portion (30) to the narrow portion (32) by transverse movement of the needle (80).

5. The handheld tracheostomy guide (1) according to any of the proceeding claims, wherein the channel (24) comprises an open portion (34) in which the needle (80) guided by the channel (24) is exposed on the front side (16) of the support body (10).

6. The handheld tracheostomy guide (1) according to any of the proceeding claims, wherein the channel comprises at least a covered portion (36) for covering the tracheostomy needle (80).

7. The handheld tracheostomy guide (1) according to any of the proceeding claims, wherein the support body (10) comprises a tubular portion (38) at the distal end (14), and wherein the tubular portion (38) forms at least a part of the channel (24).

8. The handheld tracheostomy guide (1) according to any of the proceeding claims, wherein the distal end (14) of the support body (10) comprises a contact edge (40) for contacting the throat of the patient.

9. The handheld tracheostomy guide (1) according to any of the proceeding claims, wherein the first side (20) and the second side (22) each has a proximal concave portion (44).

10. The handheld tracheostomy guide (1) according to any of the proceeding claims wherein the support body (10) has a manual and/or mechanical interface (82) on the rear side (18) and at the proximal end (12), and wherein the interface (82) is a protrusion (50) extending from the rear side (18) and forming a sector of a ring or a complete ring (90).

11. The handheld tracheostomy guide (1) according to claim 10, further comprising a tubular body (54) attached, to the support body (10), and wherein the tubular body (54) has a rear end (58) and a forward end (60), and wherein
the tubular body (54) has an aperture (56) at the rear end (58),
and wherein the tubular body (54) is configured for receiving a finger of the user through the aperture (56).

12. The handheld tracheostomy guide (1) according to claim 11, further comprising a lock (94) for releasably locking the support body (10) and the tubular body (54).

13. The handheld tracheostomy guide (1) according to claim 12, wherein the lock (94) comprises a locking member (70) pivotably connected to the tubular body (54) and configured to cooperate with support body (10).

14. The handheld tracheostomy guide (1) according to claim 13, wherein the tubular body (54) forms a first cut out (74) cooperating with the locking member (70) to pivotably connect the locking member (70) to the tubular body (54), and
wherein the rear side (18) of the support body (10) forms a second cut (76) out cooperating with the locking member (70) to releasably lock the locking member (70) to the support body (10).

15. A tracheostomy kit (100) comprising a handheld tracheostomy guide (1) according to any of the claims 1-14, and a tracheostomy needle (80) positioned in the channel (24) of the support body (10) of the handheld tracheostomy guide (1) .
